# EUROPEAN PATENT APPLICATION

(11) **EP 4 311 492 A1**
(43) Date of publication of application: **31.01.2024**
(21) Application number: 22186916.7
(22) Date of filing: 26.07.2022
(51) Int. Cl.: A61B 6/00, A61B 6/03, G06T 11/00

(54) **SPECTRAL X-RAY IMAGING**

(71) Applicant: Koninklijke Philips N.V., 5656 AG Eindhoven (NL)
(72) Inventor: WITHAGEN, Petrus, Eindhoven (NL); HUMMEL, Erik, Eindhoven (NL); VAN NIJNATTEN, Fred Simon Berend, 5656AG Eindhoven (NL); VAN DE HAAR, Peter, 5656AG Eindhoven (NL); SCHÄFER, Dirk, Eindhoven (NL)
(74) Representative: Philips Intellectual Property & Standards

(57) **Abstract**

The present invention relates to spectral imaging. In order to provide a facilitated way of spectral imaging, a method (200) for spectral X-ray imaging is provided that comprises the steps of: first rotational moving (202) of support structures of an X-ray imaging device with an X-ray source and an X-ray detector mounted to the support structures in a first movement around a region of interest of a subject; while generating (204) X-ray radiation with first X-ray spectral properties during the first rotational moving and detecting (206) a first plurality of 2D X-ray images during the first rotational moving. Further, second rotational moving (208) of the support structures in a second movement around the region of interest of a subject, wherein the second movement is generally different to the first movement; while generating (210) X-ray radiation with second X-ray spectral properties during the second rotational moving and detecting (212) a second plurality of 2D X-ray images during the second rotational moving. Reconstructing (214) a spectral 3D data set from the first plurality of 2D X-ray images and the second plurality of 2D X-ray images; and providing (216) the spectral 3D data set.

## Description

### FIELD OF THE INVENTION

The present invention relates to a device for spectral X-ray imaging, to a spectral X-ray imaging system and to a method for spectral X-ray imaging.

### BACKGROUND OF THE INVENTION

In the field of medical imaging, so-called spectral CT imaging is an extension for CT imaging, providing the user with more information. Also for the interventional laboratory, the additional information can be beneficial. As an example, spectral cone-beam CT (CBCT) can be accomplished by a dual-layer detector setup or by a dual C-arc setup. However, this results both in an expensive arrangement. The dual C-arc is possible on biplane systems. The dual layer approach requires an expensive dual-layer detector. Another option is to provide two separate scans in a dual scan mode. However, this results in an increased complexity of the workflow and increased time.

### SUMMARY OF THE INVENTION

There may thus be a need for a facilitated way of spectral imaging.

The object of the present invention is solved by the subject-matter of the independent claims; further embodiments are incorporated in the dependent claims. It should be noted that the following described aspects of the invention apply also for the device for spectral X-ray imaging, for the spectral X-ray imaging system and for the method for spectral X-ray imaging.

According to the present invention, a device for spectral X-ray imaging is provided. The device comprises a data input, a data processor and an output interface. The data input is configured to receive a first plurality of 2D X-ray images and a second plurality of 2D X-ray images. The first plurality of 2D X-ray images is detected during a first rotational moving of an X-ray source and an X-ray detector in a first movement around a region of interest of a subject. X-ray radiation with first X-ray spectral properties is generated during the first movement. The second plurality of 2D X-ray images is detected during a second rotational moving of the X-ray source and the X-ray detector in a second movement around the region of interest of a subject. X-ray radiation with second X-ray spectral properties is generated during the second movement. The second X-ray spectral properties are different from the first X-ray spectral properties. The second movement is generally different to the first movement. The data processor is configured to reconstruct a spectral 3D data set from the first plurality of 2D X-ray images and the second plurality of 2D X-ray images. The output interface is configured to provide the spectral 3D data set.

According to an example, a controller is provided. The controller is configured to operate an X-ray imaging system with an X-ray imaging device with an X-ray source and an X-ray detector mounted to movable support structures to perform the generation of the X-ray radiation with the first X-ray spectral properties while causing the X-ray source and the X-ray detector to move in the first rotational moving and to perform the generation of the X-ray radiation with the second X-ray spectral properties while causing the X-ray source and the X-ray detector to move in the second rotational moving.

As an effect, an efficient, fast and facilitated way of providing spectral imaging is provided.

In another example, a device for spectral X-ray imaging is provided. The device comprises a controller, a data input, a data processor and an output interface. The controller is configured to operate an X-ray imaging system with an X-ray imaging device with an X-ray source and an X-ray detector mounted to movable support structures to perform the steps of: first rotational moving of the X-ray source and the X-ray detector in a first movement around a region of interest of a subject; generating X-ray radiation with first X-ray spectral properties during the first movement; and detecting a first plurality of 2D X-ray images during the first rotational moving; second rotational moving of the X-ray source and an X-ray detector in a second movement around the region of interest of a subject, wherein the second movement is generally different to the first movement; and generating X-ray radiation with second X-ray spectral properties during the second movement; wherein the second X-ray spectral properties are different from the first X-ray spectral properties; and detecting a second plurality of 2D X-ray images during the second rotational moving. The data input is configured to receive the first plurality of 2D X-ray images and the second plurality of 2D X-ray images. The data processor is configured to reconstruct a spectral 3D data set from the first plurality of 2D X-ray images and the second plurality of 2D X-ray images. The output interface is configured to provide the spectral 3D data set.

According to an example, to reconstruct the spectral 3D data set, the data processor is configured to reconstruct a first volume from the first plurality of 2D X-ray images; to reconstruct a second volume from the second plurality of 2D X-ray images; and to combine the first volume and the second volume providing the spectral 3D data set.

According to an example, the data processor comprises a spectral cone beam CT reconstructor configured to reconstruct the first and the second volume from the first and second plurality of X-ray images by computed tomography.

According to an example, for combining the first volume and the second volume, the data processor is configured to at least one of the group of: i) alignment of the first volume and the second volume and ii) registration of the first volume and the second volume.

According to an example, the controller is configured to operate the X-ray imaging system to perform the first and the second rotational moving of the X-ray source and the X-ray detector as at least one of the group of a wiper motion, a closed dual-axis trajectory and a saddle trajectory.

According to an example, the data processor is configured to provide a step of material decomposition to obtain spectral information.

According to the present invention, also a spectral X-ray imaging system is provided. The system comprises an X-ray imaging device with an X-ray source and an X-ray detector mounted to movable support structures. The system also comprises a device for spectral X-ray imaging according to one of the preceding examples. The controller of the device for spectral X-ray imaging is configured to operate the support structures, the X-ray source and the X-ray detector. The X-ray imaging device is configured to provide the first and the second pluralities of 2D X-ray images.

According to an example, a display is provided configured to display the spectral 3D data set.

According to an example, the X-ray detector is provided as a dual-layer detector configured for the first and the second X-ray spectral properties.

According to the present invention, also a method for spectral X-ray imaging is provided. The method comprises the following steps: first rotational moving of support structures of an X-ray imaging device with an X-ray source and an X-ray detector mounted to the support structures in a first movement around a region of interest of a subject; generating X-ray radiation with first X-ray spectral properties during the first rotational moving; and detecting a first plurality of 2D X-ray images during the first rotational moving; second rotational moving of the support structures in a second movement around the region of interest of a subject, wherein the second movement is generally different to the first movement; generating X-ray radiation with second X-ray spectral properties during the second rotational moving; and detecting a second plurality of 2D X-ray images during the second rotational moving; reconstructing a spectral 3D data set from the first plurality of 2D X-ray images and the second plurality of 2D X-ray images; and providing the spectral 3D data set.

According to an aspect, two different measurements, i.e. two X-ray imaging runs, also referred to as scans, are provided for achieving different information. Providing the two scans as opposite directed movements allows an acquisition with the two runs as close as possible. The term "close" is meant in a timewise manner. By using a C-arm, the two X-ray imaging runs can be provided as rather fast scans, as such is possible with today's C-arm setups.

According to an aspect, a C-arc imaging system is combined with a spectral CBCT reconstructor providing spectral imaging. This can be implemented on any C-arc system without hardware changes and has a scan time of only two times the normal scan time.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for spectral X-ray imaging.
Fig. 2 shows an example of a spectral X-ray imaging system.
Fig. 3 shows basic steps of an example of a method for spectral X-ray imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

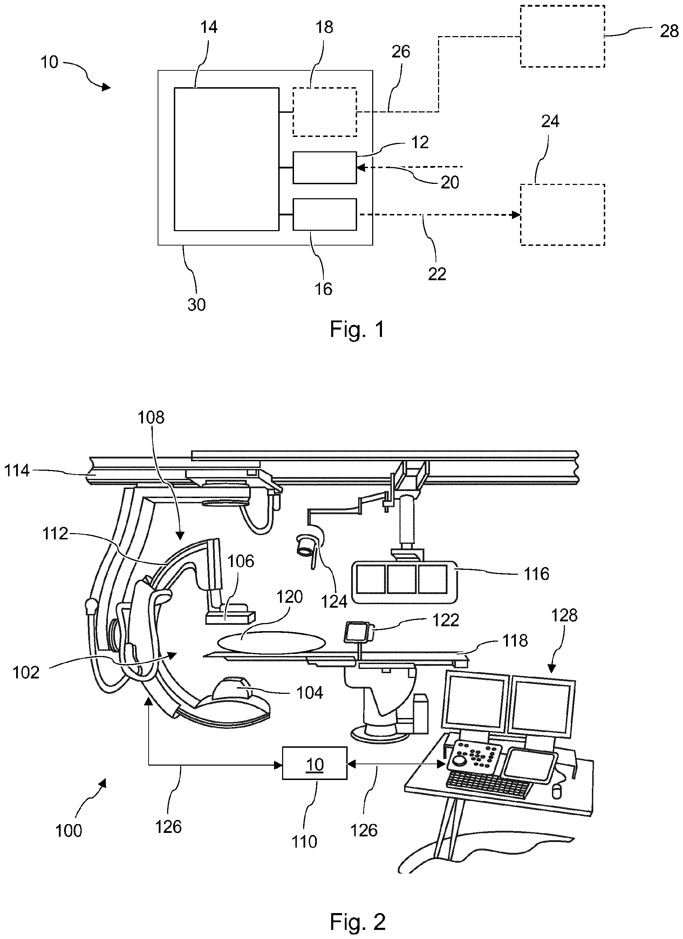

Fig. 1 schematically shows an example of a device 10 for spectral X-ray imaging. The device 10 comprises a data input 12, a data processor 14 and an output interface 16. The data input 12 is configured to receive a first plurality of 2D X-ray images and a second plurality of 2D X-ray images. The first plurality of 2D X-ray images is detected during a first rotational moving of an X-ray source and an X-ray detector in a first movement around a region of interest of a subject. X-ray radiation with first X-ray spectral properties is generated during the first movement. The second plurality of 2D X-ray images is detected during a second rotational moving of the X-ray source and the X-ray detector in a second movement around the region of interest of a subject. X-ray radiation with second X-ray spectral properties is generated during the second movement. The second X-ray spectral properties are different from the first X-ray spectral properties. The second movement is generally different to the first movement. The data processor 14 is configured to reconstruct a spectral 3D data set from the first plurality of 2D X-ray images and the second plurality of 2D X-ray images. The output interface 16 is configured to provide the spectral 3D data set.

As an option, a controller 18 (indicated with a broken line frame) is provided. The controller 18 is configured to operate an X-ray imaging system with an X-ray imaging device with an X-ray source and an X-ray detector mounted to movable support structures to:
- perform the generation of the X-ray radiation with the first X-ray spectral properties while causing the X-ray source and the X-ray detector to move in the first rotational moving; and
- perform the generation of the X-ray radiation with the second X-ray spectral properties while causing the X-ray source and the X-ray detector to move in the second rotational moving.

A first arrow 20, shown in broken line as well, indicates the data provision to the data input 12, like the provision of the first plurality of 2D X-ray images and the second plurality of 2D X-ray images.

A second arrow 22 in broken lines indicates the generated data provided to the user, for example via a display 24 (also shown in broken lines).

A first broken line 26 indicates a signal connection to an X-ray imaging system 28 with an X-ray imaging device having an X-ray source and an X-ray detector mounted to movable support structures.

A further frame 30 in broken line indicates that the data input 12, the data processor 14 and the output interface 16 can be arranged in an integrated manner, such as in a common structure or the like. As an option, also the controller 18 can be integrated. In another option, one or a few of the components are provided separately.

The term "rotational" is used as a general term here. It refers to e.g. a circular trajectory, but also to a dual-axis trajectory.

The term "first movement" can also be referred to as first direction or as first trajectory. The term "second movement" can also be referred to as second direction or as second trajectory.

In an example, the second movement is generally opposite to the first movement. For example, the trajectory may be the same but the direction is different.

The term "controller 18" relates to e.g. a processor or part of a data processor that is capable of providing control signals for the operation of an X-ray system, e.g. comprising support structures, like a C-arm or robot arms, and an X-ray imaging device with an X-ray tube and an X-ray detector. As an example, the controller 18 provides signals that result in a respective movement of the support structures arm. Further, the controller 18 provides signals that result in a respective generation of X-ray radiation by an X-ray tube and a detection of X-ray radiation by an X-ray detector.

The term "data input 12" relates to providing or supplying data for data processing steps. The data input 12 can also be referred to as image data input 12. The data input 12 can also be referred to as data supply, as image data supply, as image input, as input unit or simply as input. In an example, the image data input 12 is data-connectable to an imaging source arrangement.

The term "data processor 14" relates to a processor or part of a processor arrangement that is provided to conduct the computing steps using the data supplied by the data input 12. The data processor 14 can also be referred to as data processing arrangement, as processor unit or as processor. In an example, the data processor 14 is data-connected to the data input 12 and the output interface 16.

The term "output interface 16" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller 18 can be provided by the output interface 16.

The term "rotational moving" relates to moving the support structures around an isocenter in a way that the X-ray source and the X-ray detector are commonly moved along an arc-shaped trajectory. In an example, arc-shaped relates to a part of a circular trajectory. For example, a trajectory of 180° plus fan angle of the radiation is provided. The support structure, such as a C-arm, is rotated around the isocenter. As an example, a region of interest of a subject is arranged aligned with the isocenter.

The term "X-ray spectral properties " relates to the spectral characteristics of the respective radiation. The first and second X-ray spectral properties are set such that they are distinct to each other. For example, the first and second X-ray spectral properties are caused by two different and distinct energy levels.

The spectral 3D data can also be referred to as dual spectral 3D data.

In an example, the first movement comprises a first direction and the second movement comprises a second direction. The second direction is generally opposite to the first direction. In another example, the system keeps rotating. In a further example, a backward direction with another trajectory is provided.

In an example, for reconstructing a spectral 3D data set from the first plurality of 2D X-ray images and the second plurality of 2D X-ray images, the data processor 14 is configured to at least one of the group of: i) alignment of the first plurality of 2D X-ray images and the second plurality of 2D X-ray images, and
ii) registration of the first plurality of 2D X-ray images and the second plurality of 2D X-ray images.

The first plurality of X-ray images can also be referred to as first plurality of first X-ray images or as plurality of first X-ray images. The second plurality of X-ray images can also be referred to as second plurality of first X-ray images or as plurality of second X-ray images.

In an example, the data processor 14 comprises a spectral cone beam CT reconstructor configured to reconstruct the spectral 3D data set from the first and second plurality of X-ray images by computed tomography.

In an example, provided as an option but not shown in further detail, to reconstruct the spectral 3D data set, the data processor 14 is configured to reconstruct a first volume from the first plurality of 2D X-ray images; to reconstruct a second volume from the second plurality of 2D X-ray images; and to combine the first volume and the second volume providing the spectral 3D data set.

The first volume can also be referred to as first spectral volume. The second volume can also be referred to as second spectral volume.

The term "to reconstruct a volume" relates to computing 3D image data based on the pluralities of 2D X-ray images. The 3D image data each provide a spectral volume or spectral 3D dataset.

The term "to combine the first and second volume" relates to computing further 3D image data that comprises the information of both spectral 3D datasets.

In an example, provided as an option but not shown in further detail, the data processor 14 comprises a spectral cone beam CT reconstructor configured to reconstruct the first and the second volume from the first and second plurality of X-ray images by computed tomography.

The term "spectral cone beam CT reconstructor" relates to a processor or part of a processor that is configured to reconstruct spectral X-ray images generated with cone-beam shaped radiation.

In an example, provided as an option but not shown in further detail, for combining the first volume and the second volume, the data processor 14 is configured to at least one of the group of: i) alignment of the first volume and the second volume; and ii) registration of the first volume and the second volume.

The term "alignment" relates to arranging the first and second volume in a known relation to each other. For example, the first and second volume are arranged in a spatially matching way.

The term "registration" relates to determining the relative arrangement of the first and second volume and performing a spatial transformation of at least one of the two volumes such that the first and second volume are arranged in a spatially matching way. The registration can comprise rigid registration and elastic registration. The transformation can comprise shifting and warping of at least one of the volumes.

In an example, the acquisition is based on a forward and a backward scan, which are followed immediately one after the other with a minimal time delay of e.g. less than two seconds.

In an example, the two volumes are registered with a 3D-3D registration algorithm.

In an example, a minimum time delay of less than two seconds is provided between the first and the second rotational moving.

In an example, for reconstructing the first and the second volume, the data processor 14 is configured to take the corresponding geometry calibration into account.

In an example, provided as an option but not shown in further detail, the controller 18 is configured to operate the X-ray imaging system to perform the first and the second rotational moving of the X-ray source and the X-ray detector as a wiper motion.

In another example, provided additionally or alternatively, the controller 18 is configured to operate the X-ray imaging system to perform the first and the second rotational moving of the X-ray source and the X-ray detector as a closed dual-axis trajectory.

In a further example, provided additionally or alternatively, the controller 18 is configured to operate the X-ray imaging system to perform the first and the second rotational moving of the X-ray source and the X-ray detector as a saddle trajectory.

The wiper motion, also referred to as wiper acquisition, is a combination of two consecutive scans, one in forward and one in backward direction. The two scans each have different X-ray parameters (kV, mA, ms, filtration). In an example, the first and second scans are arranged along the same trajectory, just in different directions.

In another example, the first and second scans are provided along trajectories that are basically aligned to each other while deviating in at least certain parts.

In another example, the first and second scans are provided along trajectories that are different. For example, the trajectories are mirrored. In an option, a smooth transition is provided between the two trajectories, e.g. resulting in no need for a full stop, which means that lower forces are required.

As an example, a wiper motion is provided with a small curved trajectory end-segments to result in a smoother transition when turning backwards. Same can be provided for both turning sections, i.e. also for the starting section.

The closed dual-axis trajectory is also referred to as butterfly trajectory. The term "closed" refers to the loop-like trajectory,

During the first half of the trajectory, the first tube settings are applied and during the second half of the trajectory the second tube settings are applied. This has the advantage that no turning point is existent and hence the delay in the turning point can be avoided and total scan time is minimized. The "turns" of a saddle trajectory avoid the need for a sudden stop and a respective sudden acceleration when changing from the first to the second scan. the end

In an example, provided as an option but not shown in further detail, for achieving the first and second X-ray spectral properties, the controller 18 is configured to provide a first and a second setting. The controller 18 is configured to modify between the first and the second setting at least one of the group of tube parameters that influence the spectral properties of the X-ray beam, filtration and frame rate.

In an example, an amount of copper filtration is provided, such as 0, 0.1, 0.4 or 0.9 mm thick, but also Tin and other materials are used in further examples.

The modification of the frame rate may be useful to have a very fast scan at high kVp, where the tube my generate enough flux in short time, e.g. 120kVp, 0.4 Cu, 4s scan at 120fps, and a slower low kVp scan, e.g. 80kVp, 0.1 Cu, 7s scan at 60fps.

In an example, provided as an option but not shown in further detail, for reconstructing the spectral 3D data set, the data processor 14 is configured to provide the step of separate correction for at least one of the group of: scatter, intra-scan vibration, patient motion, noise reduction, artifact reduction and beam hardening effects.

In an example, for reconstructing the first and the second volume, the data processor 14 is configured to provide the step of separate correction for at least one of the group of: scatter, intra-scan vibration, patient motion, noise reduction, artifact reduction and beam hardening effects.

In an example, provided as an option but not shown in further detail, the data processor 14 is configured to provide a step of material decomposition to obtain spectral information.

In an example, virtual non-contrast (VNC) images and iodine maps (INW) are provided.

In an example, the data processor 14 is configured to provide a step of volume-based material decomposition to obtain spectral information

In an example, provided as an option but not shown in further detail, as a third scan with a third spectral settings, the controller 18 is configured to perform the steps of:
- third rotational moving of the support structures in the first or second movement around the region of interest of a subject; and
- generating X-ray radiation with third X-ray spectral properties during the third rotational moving; and detecting a third plurality of 2D X-ray images during the third rotational moving.

In an example, at least one further scan is provided with at least one further spectral settings.

Fig. 2 shows an example of a spectral X-ray imaging system 100. The spectral X-ray imaging system 100 comprises an X-ray imaging device 102 with an X-ray source 104 and an X-ray detector 106 mounted to movable support structures 108. The system 10 further comprises an example 110 of the device 10 for spectral X-ray imaging according to one of the preceding examples. The controller 18 of the device for spectral X-ray imaging is configured to operate the support structures 108, the X-ray source 104 and the X-ray detector 106. The X-ray imaging device 102 is configured to provide the first and the second pluralities of 2D X-ray images.

The term "support structures" relates to different ways of supporting, i.e. mounting the X-ray source and the X-ray detector. The support structures 108 are movably to allow a movement of the X-ray source 104 and the X-ray detector 106 in relation to a subject.

The controller 18 is configured to operate the X-ray imaging device 102.

In an example, the X-ray source 104 and the X-ray detector 106 are supported by, i.e. mounted to, individual arms that can be moved around a subject such that X-ray source 104 and the X-ray detector 106 are arranged in predetermined relation to each other, such as keeping their distance and orientation to each other. The arms can be provided as separate robot arms.

In an example, depicted in Fig. 2 as an option, the X-ray source 104 and the X-ray detector 106 are mounted to a movably mounted C-arm 112. For example, the X-ray source 104 and the X-ray detector 106 are mounted to opposing ends of the C-arm 112.

In an example, shown in Fig. 2 as an option, the spectral X-ray imaging system 100 is provided as a stationary system, e.g. a system fixedly mounted in an operation room, cathlab or the like. For example, the C-arm 112 is movably attached to a ceiling mounted rail system 114. As another example, the C-arm 112 is movably attached to a floor- or wall-mounted base.

In another example, the spectral X-ray imaging system 100 is provided as a mobile system. For example, the C-arm 112 is movably attached to a mobile base, such as a base structure with wheels.

As an option, Fig. 2 shows a display 116 that is configured to display the spectral 3D data set.

The display 116 may be part of a cathlab setup that also comprises a subject table 118 on which a subject 120 can be arranged. A control interface 122 is shown plus lighting equipment 124. A first data connection line 126 indicates the data-connection of the example 110 of the device 10 for spectral X-ray imaging to the X-ray imaging system 102.

As a further option, a control or operating console 128 is shown in the right foreground. According to an option, the console 128 is data-connected to the example 110 of the device 10 for spectral X-ray imaging, as indicated with second data connection line 130

In an example, provided as an option but not shown in further detail, the X-ray detector 106 is provided as a dual-layer detector configured for the first and the second X-ray spectral properties.

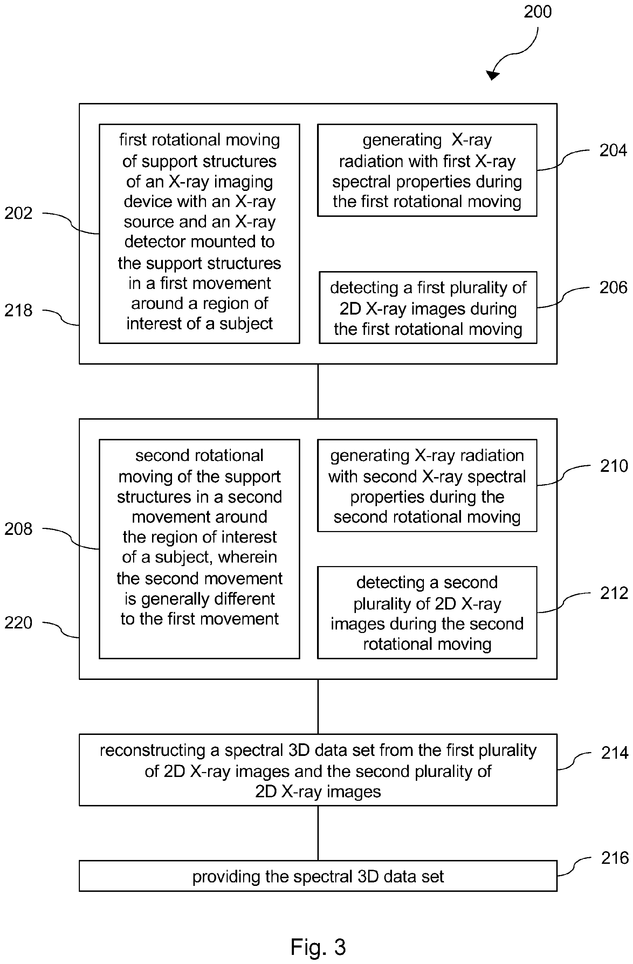

The dual-layer detector improves the spectral separation power of the imaging procedure. Fig. 3 shows basic steps of an example of a method 200 for spectral X-ray imaging. The method 200 comprises the following steps:
In a first sub-step 202 of a first imaging step, it is provided first rotational moving of support structures of an X-ray imaging device with an X-ray source and an X-ray detector mounted to the support structures in a first movement around a region of interest of a subject.

In a second sub-step 204 of the first imaging step, it is provided generating of X-ray radiation with first X-ray spectral properties during the first rotational moving.

In a third sub-step 206 of the first imaging step, it is provided detecting a first plurality of 2D X-ray images during the first rotational moving.

In a first sub-step 208 of a second imaging step, it is provided second rotational moving of the support structures in a second movement around the region of interest of a subject. The second movement is generally different to the first movement.

In a second sub-step 210 of a second imaging step, it is provided generating X-ray radiation with second X-ray spectral properties during the second rotational moving

In a third sub-step 212 of a second imaging step, it is provided detecting a second plurality of 2D X-ray images during the second rotational moving.

In a further step 214, it is provided reconstructing a spectral 3D data set from the first plurality of 2D X-ray images and the second plurality of 2D X-ray images.

In a still further step 216, it is provided providing the spectral 3D data set. A first frame 218 indicates the first imaging step or first imaging procedure, also referred to as acquiring the first plurality of 2D X-ray images.

A second frame 220 indicates the second imaging step or second imaging procedure, also referred to as acquiring the second plurality of 2D X-ray images.

The (sub-) step of the first rotational moving and the (sub-) step of the generating of the first radiation, i.e. radiation with first X-ray spectral properties, and the (sub-) step of detecting of the first plurality of X-ray images take place at basically the same time.

The (sub-) step of the second rotational moving and the (sub-) step of the generating of the second radiation, i.e. radiation with second X-ray spectral properties, and the (sub-) step of detecting of the second plurality of X-ray images take place at basically the same time.

As an option, step 216 comprises displaying the spectral 3D data set.

In an example of the method, for reconstructing the spectral 3D data set, it is provided the steps of:
- reconstructing a first spectral volume from the first plurality of 2D X-ray images and reconstructing a second volume from the second plurality of 2D X-ray images; and
- combining the first volume and the second volume providing the spectral 3D data set.

In an example, the first rotational moving, the generating X-ray radiation with first X-ray spectral properties and the detecting of the first plurality of 2D X-ray images takes place more or less simultaneously as a first scan. The second rotational moving, the generating X-ray radiation with second X-ray spectral properties and the detecting of the second plurality of 2D X-ray images takes place more or less simultaneously as a second scan.

The first rotational moving can be referred to as forward scan and the second rotational moving can be referred to as backward scan.

In an example of the method, the first and the second rotational moving are provided as a wiper motion.

In an example of the method, the first and the second rotational moving are provided as a closed dual-axis trajectory.

In an example of the method, for combining the first volume and the second volume, it is provided at least one step of the group of:
- aligning the first volume and the second volume; and
- registering the first volume and the second volume.

In an example of the method, for reconstructing the first and the second volume, the corresponding geometry calibration is taking into account.

In an example of the method, for achieving the first and second X-ray spectral properties, it is provided a first and a second setting, wherein it is provided modifying between the first and the second setting at least one of the group of:
- tube parameters that influence the spectral properties of the X-ray beam;
- frame rate.

In an example of the method, for reconstructing the first and the second volume, it is provided the step of separately correcting for at least one of the group of scatter, intra-scan vibration, patient motion and beam hardening effects.

In an example of the method, it is further provided a step of:
- volume based material decomposition to obtain spectral information

In an example of the method, as a third scan with a third spectral settings, it is provided the steps of:
- third rotational moving of a support structures, e.g. a C-arm, in the first or second movement around the region of interest of a subject;
- generating X-ray radiation with third X-ray spectral properties during the third rotational moving; and detecting a third plurality of 2D X-ray images during the third rotational moving.

In an example of the method, the steps of reconstruction and combining, e.g. the registration, are combined such that after an initial reconstruction and 3D-3D registration, a unified geometry is used for any further processing of scatter, vibration and patient motion correction. As an option, the unified geometry is used to apply projection based material decomposition.

In another example, the following is provided: A first scan is performed with first settings of tube, e.g. 120kVp, 0.4 mm Cu-filtration, 60 fps), and along a trajectory of e.g. a circular scan 180° +fan-angle. As an option, a minimum time delay of less than 2 seconds between the scans is provided. A second scan is performed with second settings to exploit spectral information from both scans with respect to the X-ray tube, e.g. 80kVp, 0.1 mm Cu-filtration, 60 fps, and along a trajectory of e.g. a circular scan in reverse direction. Each of the scans is reconstructed separately, optionally taking into account the corresponding geometry calibration and correcting separately for scatter, intra-scan vibration and patient motion if necessary as well as (water and bone) beam hardening effects. The two volumes are registered, e.g. with a 3D-3D registration algorithm. A volume based material decomposition is performed to obtain spectral information, e.g. Virtual Non-Contrast (VNC) images and Iodine Maps (INW) .

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system. The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

In an example, a computer program or program element for controlling an apparatus according to one of the examples above is provided, which program or program element, when being executed by a processing unit, is adapted to perform the method steps of one of the method examples above.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for spectral X-ray imaging, the device comprising:
- a data input (12);
- a data processor (14); and
- an output interface (16);
wherein the data input is configured to receive a first plurality of 2D X-ray images and a second plurality of 2D X-ray images;
wherein the first plurality of 2D X-ray images is detected during a first rotational moving of an X-ray source and an X-ray detector in a first movement around a region of interest of a subject; wherein X-ray radiation with first X-ray spectral properties is generated during the first movement;
wherein the second plurality of 2D X-ray images is detected during a second rotational moving of the X-ray source and the X-ray detector in a second movement around the region of interest of a subject; wherein X-ray radiation with second X-ray spectral properties is generated during the second movement; wherein the second X-ray spectral properties are different from the first X-ray spectral properties; and wherein the second movement is generally different to the first movement;
wherein the data processor is configured to reconstruct a spectral 3D data set from the first plurality of 2D X-ray images and the second plurality of 2D X-ray images; and
wherein the output interface is configured to provide the spectral 3D data set.

2. Device according to claim 1, further comprising:
- a controller (18);
wherein the controller is configured to operate an X-ray imaging system with an X-ray imaging device with an X-ray source and an X-ray detector mounted to movable support structures to:
- perform the generation of the X-ray radiation with the first X-ray spectral properties while causing the X-ray source and the X-ray detector to move in the first rotational moving; and
- perform the generation of the X-ray radiation with the second X-ray spectral properties while causing the X-ray source and the X-ray detector to move in the second rotational moving.

3. Device according to claim 1 or 2, wherein, to reconstruct the spectral 3D data set, the data processor is configured to reconstruct a first volume from the first plurality of 2D X-ray images; to reconstruct a second volume from the second plurality of 2D X-ray images; and to combine the first volume and the second volume providing the spectral 3D data set.

4. Device according to claim 3, wherein the data processor comprises a spectral cone beam CT reconstructor configured to reconstruct the first and the second volume from the first and second plurality of X-ray images by computed tomography.

5. Device according to claim 3 or 4, wherein, for combining the first volume and the second volume, the data processor is configured to at least one of the group of:
i) alignment of the first volume and the second volume; and
ii) registration of the first volume and the second volume.

6. Device according to one of the preceding claims, wherein the controller is configured to operate the X-ray imaging system to perform the first and the second rotational moving of the X-ray source and the X-ray detector as at least one of the group of:
- a wiper motion;
- a closed dual-axis trajectory; and
- a saddle trajectory.

7. Device according to one of the preceding claims, wherein, for achieving the first and second X-ray spectral properties, the controller is configured to provide a first and a second setting, wherein the controller is configured to modify between the first and the second setting at least one of the group of:
- tube parameters that influence the spectral properties of the X-ray beam;
- filtration; and
- frame rate.

8. Device according to one of the preceding claims, wherein, for reconstructing the spectral 3D data set, the data processor is configured to provide the step of separate correction for at least one of the group of: scatter, intra-scan vibration, patient motion, noise reduction, artifact reduction and beam hardening effects.

9. Device according to one of the preceding claims, wherein the data processor is configured to provide a step of material decomposition to obtain spectral information.

10. Device according to one of the preceding claims, wherein, as a third scan with a third spectral settings, the controller is configured to perform the steps of:
- third rotational moving of the support structures in the first or second movement around the region of interest of a subject; and
- generating X-ray radiation with third X-ray spectral properties during the third rotational moving; and detecting a third plurality of 2D X-ray images during the third rotational moving.

11. A spectral X-ray imaging system (100), comprising:
- an X-ray imaging device (102) with an X-ray source (104) and an X-ray detector (106) mounted to movable support structures (108); and
- a device (110) for spectral X-ray imaging according to one of the preceding claims;
wherein the controller of the device for spectral X-ray imaging is configured to operate the support structures, the X-ray source and the X-ray detector; and
wherein the X-ray imaging device is configured to provide the first and the second pluralities of 2D X-ray images.

12. System according to claim 11, wherein a display (116) is provided configured to display the spectral 3D data set.

13. System according to claim 11 or 12, wherein the X-ray detector is provided as a dual-layer detector configured for the first and the second X-ray spectral properties.

14. A method (200) for spectral X-ray imaging, comprising the following steps:
- first rotational moving (202) of support structures of an X-ray imaging device with an X-ray source and an X-ray detector mounted to the support structures in a first movement around a region of interest of a subject;
- generating (204) X-ray radiation with first X-ray spectral properties during the first rotational moving; and detecting (206) a first plurality of 2D X-ray images during the first rotational moving;
- second rotational moving (208) of the support structures in a second movement around the region of interest of a subject, wherein the second movement is generally different to the first movement;
- generating (210) X-ray radiation with second X-ray spectral properties during the second rotational moving; and detecting (212) a second plurality of 2D X-ray images during the second rotational moving;
- reconstructing (214) a spectral 3D data set from the first plurality of 2D X-ray images and the second plurality of 2D X-ray images; and
- providing (216) the spectral 3D data set.

15. Computer program comprising instructions which, when the program is executed by a computer, cause the computer to carry out the method of claim 14.
